# FASCICULE DE BREVET EUROPEEN

(11) **EP 0 843 538 B1**
(45) Date de publication et mention de la délivrance du brevet: **12.06.2002**
(21) Numéro de dépôt: 95939332.3
(22) Date de dépôt: 07.11.1995
(51) Int. Cl.: A61F 2/06

(54) **MANCHON EXTENSIBLE INTERNE A USAGE CHIRURGICAL POUR DILATATION DE CONDUITS PHYSIOLOGIQUES**
INTERNE AUSDEHNBARE MANSCHETTE ZUM CHIRURGISCHEN GEBRAUCH ZUR DEHNUNG PHYSIOLOGISCHER GEFÄSSE
STENT FOR EXPANDING PHYSIOLOGICAL VESSELS

(30) Priorité: 16.12.1994 FR 9415527
(43) Date de publication de la demande: 27.05.1998
(73) Titulaire: Fouere, Alain, 13008 Marseille (FR)
(72) Inventeur: Fouere, Alain, 13008 Marseille (FR)
(74) Mandataire: Roman, Michel
(86) Numéro de dépôt international: FR9501460
(87) Numéro de publication internationale: WO9618359

(56) Documents cités:
- EP-A- 0 221 570
- EP-A- 0 335 341
- WO-A-92/06734
- WO-A-95/09584
- US-A- 5 104 404
- US-A- 5 397 355

## Description

La présente invention a pour objet un manchon extensible interne à usage chirurgical pour dilatation de conduits physiologiques comme défini dans le préambule de la revendication 1. Un tel manchon est connu du document EP-A-0 221 570 ou du document EP-A-0 335 341.

Elle est avant tout destinée à être mise en place dans une artère ou une veine pour empêcher localement son écrasement afin de permettre le passage du sang.

Ce type de prothèse est en soi connu et utilisé depuis plusieurs années. Il en existe plusieurs modèles, fabriqués en faisant appel à des techniques variées.

A titre d'exemple, le brevet européen N° EP 221 570, déposé par M. Julio Palmaz, décrit une prothèse vasculaire expansible intraluminaire, comprenant un organe de forme tubulaire à paroi mince formée par une pluralité d'éléments allongés s'intersectant l'un avec l'autre, cet élément tubulaire présentant un diamètre permettant de le faire passer à l'intérieur de la section de passage du conduit récepteur, ce diamètre pouvant être augmenté par l'application, par l'intérieur de l'élément tubulaire, d'une force s'étendant radialement vers l'extérieur, le diamètre obtenu étant variable et dépendant de l'ampleur de la force appliquée à l'organe tubulaire, certains des éléments allongés étant déformés de façon définitive par la force exercée radialement vers l'extérieur pour amener l'élément tubulaire à son diamètre expansé, ce par quoi l'élément tubulaire peut dilater la section de passage du conduit et demeurer dans cet état.

A notre connaissance, aucun dispositif de ce type ne comporte de moyens satisfaisants assurant leur maintien en place, et il arrive trop fréquemment qu'ils soient entraînés sur une certaine distance par le flot sanguin, ce qui nécessite souvent une intervention chirurgicale. Le seul moyen de garantir à long terme le maintien en place de l'élément dilatateur, est d'exercer une force d'expansion élevée, ce qui entraîne le risque de provoquer la rupture du vaisseau traité, d'autant plus que ce vaisseau est dans la plupart des cas fragilisé par l'âge ou par la maladie.

Le brevet international N° WO 92 06 734 déposé par M. Ho Young SONG fait état d'une prothèse destinée à élargir le passage de conduits physiologiques, constituée d'une structure cylindrique auto-extensible radialement, réalisée en fil métallique soudé. La prothèse comprend une pluralité d'unités circulaires fermées en forme de "zigzag" agencées de façon à former un cylindre et incluant chacune une série sans fin de sections droites reliées par des "noeuds". Ces unités circulaires sont rendues solidaires les unes des autres par des éléments de liaison périphériques longitudinaux disposés en quinconce ou en diagonale, également en fil métallique soudé, certains de ces éléments comportant des "barbes" anti-migration, faisant saillie vers l'extérieur. La paroi externe de la prothèse est recouverte d'un filet assurant l'étanchéité de l'ensemble.

La mise en place est effectuée en comprimant la prothèse pour l'introduire dans un tube monté à l'extrémité d'un cathéter, l'élasticité du matériau constitutif assurant son extension radiale dès qu'elle est libérée.

Ce dispositif présente un certain nombre d'inconvénients. L'auto-extension ne permet pas de contrôler la pression qu'il exerce sur la face interne du conduit, et les "barbes" anti-migratoires en fil métallique risquent de blesser ou même de perforer la paroi de ce dernier. En outre le filet d'étanchéité, disposé à l'extérieur de la prothèse risque également d'être percé par les "barbes" anti-migration.

Les manchons extensibles tels que ceux décrits ci-dessus comportent en outre le risque de se cintrer une fois mis en place, ce qui peut compromettre le succès de l'intervention dans un certain nombre d'interventions.

Le dispositif selon la présente invention supprime tous ces inconvénients. En effet, il permet d'obtenir des éléments extensibles ne présentant aucun risque de migration ou de cintrage non contrôlé, tout en nécessitant une force d'expansion réduite pour sa mise en place et en exerçant sur les parois du conduit physiologique une pression parfaitement déterminée.

Il est constitué comme défini dans la revendication 1, conformémment à une réalisation spécifique il est constitué d'un élément tubulaire cylindrique métallique à paroi mince dont la paroi comporte une série de perforations déterminant des éléments annulaires et des éléments longitudinaux alternativement déformables et non déformables constituant un manchon malléable pouvant s'étendre radialement sous l'effet d'une pression interne et garder la forme ainsi obtenue, cet élément tubulaire comportant de préférence des moyens d'accrochage non blessants intégrés, dirigés vers l'extérieur et aptes à empêcher toute migration du dispositif.

Sur les dessins annexés, donnés à titre d'exemples non limitatifs de formes de réalisation de l'objet de l'invention:
la figure 1 est une vue isométrique montrant un manchon pourvu de différents types de moyens d'accrochage, avant extension,
la figure 2 représente dans les mêmes conditions le manchon mis en place, après extension,
la figure 3 est une coupe axiale d'un manchon revêtu intérieurement d'un tube souple et monté sur un ballon gonflable destiné à assurer sa dilatation,
la figure 4 représente un manchon extensible dont les moyens d'accrochage sont constitués de cannelures annulaires,
la figure 5 montre un manchon court constitué d'une seule rangée de mailles,
la figure 6 représente, vu de côté, un manchon articulé constitué de deux éléments reliés par une bande longitunale,
la figure 7 montre le manchon de la figure précédente rendu rigide par des crochets amovibles,
la figure 8 représente un manchon articulé constitué de deux éléments reliés par un filet souple,
et la figure 9 est une représentation isométrique d'un manchon dont certaines parties sont recouvertes d'une couche de métal visible aux rayons X.

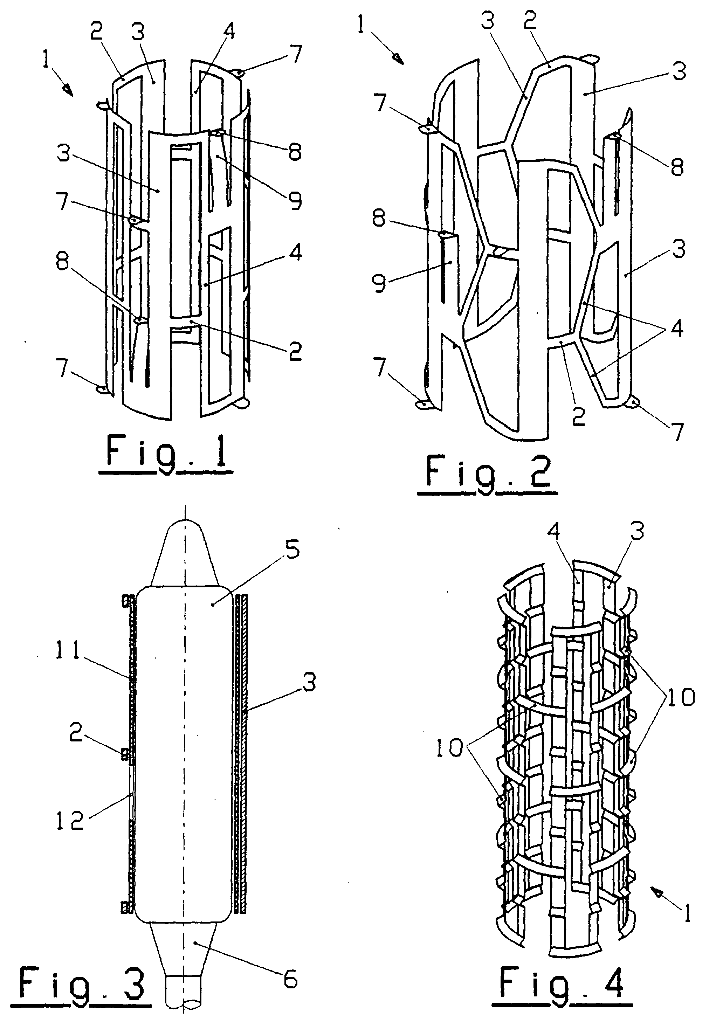

Le dispositif, figures 1 à 9, est constitué d'une gaine tubulaire 1 - ou manchon - formée d'une portion de cylindre creux dont la paroi, réalisée en matériau semi-rigide malléable, est découpée de manière à déterminer des éléments annulaires 2 et longitudinaux 3, 4 jouant le rôle de "mailles" et agencés de manière à permettre à ladite gaine de s'étendre radialement sous l'effet d'une pression interne et de garder la forme ainsi obtenue (figure 2). La pression interne est provoquée par exemple au moyen d'un élément gonflable 5 monté à l'extrémité d'un cathéter 6 (figure 3) selon une technique connue.

Le manchon sera de préférence réalisé par électro-érosion à partir d'un bloc de métal tel que l'acier inoxydable ou le tantale, ou par gravure d'un tube à paroi mince. Il sera par exemple constitué d'éléments allongés longitudinaux 3, 4 parallèles à l'axe du manchon, reliés entre eux par des éléments annulaires 2 disposés en quinconce formant ainsi des mailles rectangulaires. Des éléments longitudinaux non déformables 3 larges seront disposés alternativement avec des éléments longitudinaux 4 fins et déformables, de manière à ce que la dilatation du manchon ne modifie pas sa longueur initiale. Le manchon 1 pourra toutefois presenter dans le cadre de la revendication 1 toute configuration permettant une extension ajustable en fonction de la pression appliquée.

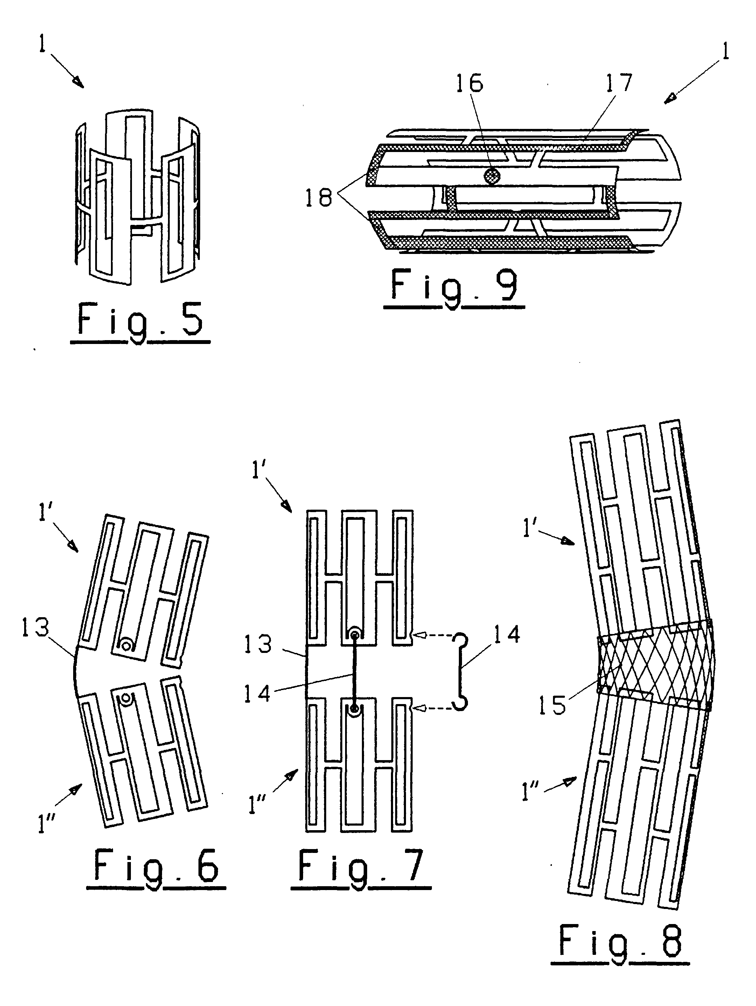

Pour certaines applications, en particulier pour le traitement des vaisseaux du cerveau, le manchon 1 doit être très court. Il sera dans ce cas constitué d'une seule rangée de mailles (figure 5).

Le manchon 1 sera avantageusement pourvu de moyens d'accrochage intégrés, dirigés radialements vers l'extérieur de façon à s'enfoncer dans la paroi interne du vaisseau sous l'effet de la pression de l'élément gonflable 5, empêchant ainsi toutes possibilités de déplacement du dispositif.

Ces moyens d'accrochage sont constitués d'éléments proéminents 7 disposés aux extrémités et/ou dans la partie courante de la gaine 1. Il peut avantageusement s'agir d'éléments proéminents rétractables 8 portés par des lames 9 incurvées vers l'intérieur du manchon, de telle manière qu'ils se trouvent normalement en retrait de la paroi de l'élément tubulaire et ne sortent de ce dernier que lorsque l'élément gonflable 5 de mise en place est sous pression.

Les éléments proéminents 7, 8, auront une forme arrondie afin de ne pas risquer de blesser la paroi du conduit physiologique et pourront être réalisés par tous moyens appropriés. Ils seront de préférence formés par pliage de parties prévues à cet effet de la paroi de la gaine 1, mais pourront aussi bien être obtenus par soudage sur ladite paroi de pièces rapportées. Ces éléments pourront éventuellement être amovibles, par exemple par cassure, de façon à permettre au chirurgien de pouvoir décider, au moment de l'opération, du nombre de moyens d'accrochage à utiliser sans avoir à disposer pour cela d'une série de manchons différents.

Dans une variante d'exécution, les moyens d'accrochage sont constitués de cannelures 10 ou ondulations annulaires de la paroi de la gaine 1, pouvant éventuellement être combinées avec des éléments proéminents obtenus par pliage ou soudage (figure 4).

Un tube souple 11 constituant un fourreau étanche pourra revêtir la face interne ou externe de la paroi du manchon 1 de manière à permettre l'utilisation du dispositif pour le traitement chirurgical de tout types d'anévrisme. Il sera réalisé en polyester (type "Dacron"®), en fibres silicones (type "Téflon"®), en polyuréthanne ou ipolyéthylène, tissé ou tricoté, imprégné ou non. Ce tube pourra être interrompu par endroits par des lacunes 12 pour permettre le passage vers des artères ou veines secondaires (figure 3).

Le manchon 1 peut être constitué de plusieurs éléments 1', 1" articulés entre eux grâce à une ou plusieurs bandes longitudinales 13 solidaires de deux éléments contigus 1', 1''. Des crochets 14, éventuellement amovibles, pourront être prévus pour bloquer une ou plusieurs articulations si nécessaire (figures 6 et 7).

Les articulations peuvent également être obtenues au moyen d'un filet 15 ou un film souple permettant au dispositif d'être placé dans une portion courbée d'un vaisseau (figure 8).

L'acier inoxydable est peu visible aux rayons X. Pour y remédier, les manchons 1 réalisés dans ce matériau comporteront avantageusement des points 16 ou des bandes longitudinales 17 ou annulaires 18 constituées de couches d'un métal lourd tel que le tantale, le titane ou l'or déposé par voie électrolytique. Ces couches pourront avoir une épaisseur voisine de 0,1 mm.

Le dispositif décrit est essentiellement destiné à traiter des vaisseaux sanguins présentant une ou plusieurs zones de rétrécissement ou de dilatation (anévrisme) localisées, mais il peut parfaitement être utilisé pour les autres conduits physiologiques, oesophage, intestins, uretères ou urètre, cholédoque, canal pancréatique, etc.

Le positionnement des divers éléments constitutifs donne à l'objet de l'invention un maximum d'effets utiles qui n'avaient pas été, à ce jour, obtenus par des dispositifs similaires.

## Revendications

1. Manchon extensible interne à usage chirurgical pour dilatation de conduits physiologiques, constitué d'un élément tubulaire cylindrique creux à paroi mince en métal malléable pouvant s'étendre radialement sous l'effet d'une pression interne d'expansion et conserver la forme ainsi obtenue, destiné en particulier à être mis en place, au moyen d'un ballon (2) gonflable, dans une artère ou une veine pour empêcher localement son rétrécissement ou son écrasement, mais pouvant être utilisé pour d'autres conduits physiologiques tels qu'oesophage, intestins, uretère, urètre, cholédoque ou canal pancréatique, la paroi mince étant découpée de manière à déterminer des éléments longitudinaux parallèles à l'axe du manchon (1) et reliés entre eux par des éléments annulaires (2) disposés en quinconce de façon à former des mailles rectangulaires,
**caractérisé en ce que** les éléments longitudinaux sont alternativement minces (4) et déformables puis larges (3) et non déformables (3).

2. Manchon extensible selon la revendication 1, se caractérisant par le fait qu'il est réalisé par électro-érosion à partir d'un bloc métallique.

3. Manchon extensible selon l'une quelconque des revendications précédentes, se caractérisant par le fait qu'il comporte des moyens d'accrochage (7, 8, 10) intégrés dirigés radialement vers l'extérieur de façon à pouvoir s'enfoncer dans la paroi interne du conduit sous l'effet de la pression d'expansion, de manière à empêcher toute migration du dispositif, lesdits moyens d'accrochage ayant une forme arrondie déterminée pour ne pas blesser la paroi du conduit physiologique.

4. Manchon extensible selon la revendication 3, se caractérisant par le fait que les moyens d'accrochage sont constitués d'éléments proéminents (7) disposés aux extrémités et/ou en partie courante du manchon (1).

5. Manchon extensible selon la revendication 3, se caractérisant par le fait que les moyens d'accrochage sont des éléments proéminents rétractables (8) portés par des lames (9) incurvées vers l'intérieur du manchon (1), de telle manière qu'ils se trouvent normalement en retrait de la paroi de l'élément tubulaire et ne sortent de ce dernier que lorsque le ballon (2) de mise en place est sous pression.

6. Manchon extensible selon l'une quelconque des revendications 4 et 5, se caractérisant par le fait que les éléments proéminents (7, 8) sont réalisés par pliage de parties de forme appropriée de la paroi du manchon (1).

7. Manchon extensible selon l'une quelconque des revendications 4 à 6, se caractérisant par le fait que les éléments proéminents (7, 8) sont amovibles.

8. Manchon extensible selon la revendication 3, se caractérisant par le fait que les moyens d'accrochage sont constitués de cannelures (10) ou ondulations annulaires de la paroi du manchon (1).

9. Manchon extensible selon l'une quelconque des revendications précédentes, se caractérisant par le fait qu'il est constitué de plusieurs éléments articulés (1', 1"), deux éléments contigus étant reliés entre eux par une bande longitudinale (13) solidaire desdits éléments.

10. Manchon extensible selon la revendication 9, se caractérisant par le fait que chaque articulation entre les éléments articulés (1', 1") peut être bloquée ou débloquée aux moyens de crochets (14) amovibles.

11. Manchon extensible selon l'une quelconque des revendications 1 à 8, se caractérisant par le fait qu'il est constitué de plusieurs éléments articulés (1', 1"), deux éléments contigus étant reliés entre eux par un filet (15) ou un film souple.

12. Manchon extensible selon l'une quelconque des revendications précédentes, se caractérisant par le fait que la face interne de la paroi du manchon (1) est revêtue d'un tube souple (11) constituant un fourreau étanche.

13. Manchon extensible selon l'une quelconque des revendications 1 à 11, se caractérisant par le fait que la face externe de la paroi du manchon (1) est revêtue d'un tube souple (11) constituant un fourreau étanche.

14. Manchon extensible selon l'une des revendications 12 ou 13, se caractérisant par le fait que le tube souple (11) est réalisé en fibres synthétiques tissées ou tricotées.

15. Manchon extensible selon l'une quelconque des revendications 12 à 14, se caractérisant par le fait que le tube souple (11) est réalisé dans l'un des matériaux suivants: polyester (genre "Dacron"), fibres silicones (genre "Téflon"), polyuréthanne ou polyéthylène.

16. Manchon extensible selon l'une quelconque des revendications 13 à 15, se caractérisant par le fait que le tube souple (11) comporte des lacunes destinées à permettre le passage vers des conduits secondaires.

17. Manchon extensible selon l'une quelconque des revendications précédentes, se caractérisant par le fait qu'il est réalisé en acier inoxydable et qu'il comporte des points (16), ou des bandes longitudinales (17) ou annulaires (18) constitués de couches d'un métal lourd visible aux rayons X, tel que le tantale, le titane ou l'or, déposé par voie électrolytique, l'épaisseur de ces couches étant voisine de 0,1 millimètre.

## Claims

1. Extensible internal sleeve for use in surgery to dilate physiological ducts, made up of a thin-walled hollow cylindrical tubular element made of malleable metal capable of extending radially under the effect of an internal expansion pressure and of retaining the form thus obtained, in particular intended to be installed by means of an inflatable balloon (2) in an artery or a vein to locally prevent it from contracting or being crushed, but which can be used in other physiological ducts such as the esophagus, intestines, ureter, urethra, choledoc or pancreatic ducts, the thin wall being cut out so as to determine the longitudinal elements parallel to the sleeve (1) axis and interconnected by the annular elements (2) in a staggered arrangement so as to form rectangular meshes,
**characterized in that** the longitudinal elements are alternatively thin (4) and deformable then broad (3) and indeformable (3).

2. Extensible sleeve according to claim 1, **characterized by** the fact that it executed by electrical discharge machining from a metal block.

3. Extensible sleeve according to any of the preceding claims, **characterized by** the fact that it comprises integrated fixing means (7, 8, 10) radially directed towards the outside so that they can be inserted in the internal wall of the duct by the expansion pressure, and thereby prevent any migration of the device, the aforementioned means of fixing having a rounded form so as not to damage the wall of the physiological duct.

4. Extensible sleeve according to claim 3, **characterized by** the fact that the fixing means consist of prominent elements (7) arranged at the ends and/or in the standard part of the sleeve (1).

5. Extensible sleeve according to claim 3, **characterized by** the fact that the fixing means consist of retractable prominent elements (8) borne on blades (9) inwardly curved towards the interior of the sleeve (1), in such a manner that their normal position is set back from the wall of the tubular element and only leave this position when the installation balloon (2) is pressurized.

6. Extensible sleeve according to any of claims 4 and 5, **characterized by** the fact that the prominent elements (7, 8) are obtained by folding parts of appropriate shape of the sleeve wall (1).

7. Extensible sleeve according to any of claims 4 to 6, **characterized by** the fact that the prominent elements (7, 8) are removable.

8. Extensible sleeve according to claim 3, **characterized by** the fact that the fixing means consist of grooves (10) or annular undulations of the sleeve wall (1).

9. Extensible sleeve according to any of the preceding claims, **characterized by** the fact that it consists of several articulated elements (1', 1"), two adjoining elements being connected by a longitudinal band (13) joined to the aforesaid elements.

10. Extensible sleeve according to claim 9, **characterized by** the fact that each articulation between the articulated elements (1', 1") can be blocked or released by means of removable hooks (14).

11. Extensible sleeve according to any of claims 1 to 8, **characterized by** the fact that it consists of several articulated elements (1', 1"), two adjoining elements being connected by a net (15) or a flexible film.

12. Extensible sleeve according to any of the preceding claims, **characterized by** the fact that the internal face of the sleeve (1) wall is lined with a flexible tube (11) to constitute a tight sleeve.

13. Extensible sleeve according to any of claims 1 to 11, **characterized by** the fact that the external face of the sleeve (1) wall is lined with a flexible tube (11) to constitute a tight sleeve.

14. Extensible sleeve according to one of claims 12 or 13, **characterized by** the fact that the flexible tube (11) is made out of woven or knitted synthetic fibers.

15. Extensible sleeve according to any of claims 12 to 14, **characterized by** the fact that the flexible tube (11) is produced in one of following materials: polyester (type "Dacron"), silicon fibers (type "Teflon"), polyurethane or polyethylene.

16. Extensible sleeve according to any of claims 13 to 15, **characterized by** the fact that the flexible tube (11) incorporates gaps to allow passage towards secondary ducts.

17. Extensible sleeve according to any of the preceding claims, **characterized by** the fact that it is made out of stainless steel and that it comprises points (16), or longitudinal (17) or annular (18) strips, consisting of layers of a heavy metal visible by x-ray examination, such as tantalum, titanium or gold, deposited by electrolysis, these layers being close to 0.1 mm thick.

## Patentansprüche

1. Innerliche, dehnbare Hülse für chirurgische Zwecke zur Erweiterung von physiologischen Kanälen, bestehend aus einem rohrförmigen, zylindrischen Element mit dünner Wandung aus einem formbaren Metall, das sich unter dem Einfluss eines Expansionsdrucks radial ausdehnen und diese Form bewahren kann, wobei die Hülse insbesondere dazu bestimmt ist, mittels eines aufblasbaren Ballons (2) in eine Arterie oder Vene eingesetzt zu werden, um deren örtliche Verengung oder Quetschung zu verhindern, die jedoch auch für andere physiologische Kanäle, wie zum Beispiel Speiseröhre, Darm, Ureter, Urethra, Gallengang oder Pankreas-Kanal geeignet ist, und deren dünne Wand derart mit Ausschnitten versehen ist, dass in Längsrichtung der Hülse parallel zur Hülsenachse (1) liegende Elemente entstehen, die untereinander durch versetzt angeordnete, ringförmige Elemente (2) verbunden sind, so dass rechteckförmige Maschen entstehen,
**gekennzeichnet dadurch, dass** die in Längsrichtung liegenden Elemente abwechselnd schmal (4) und verformbar und dann wieder breit (3) und nicht verformbar sind.

2. Dehnbare Hülse gemäß Anspruch 1, **gekennzeichnet dadurch, dass** sie per Elektroerosion aus einem vollen Metallblock gearbeitet wurde.

3. Dehnbare Hülse nach einem der beiden vorstehenden Anspruche, **gekennzeichnet dadurch, dass** sie integrierte hakenförmige Gebilde (7, 8, 10) besitzt, die radial nach außen zeigen, so dass sie unter dem Einfluss des aufweitenden Innendrucks in die Innenwand des Kanals eingreifen und so ein Wandern der Vorrichtung verhindern können, wobei die hakenförmigen Gebilde eine vorbestimmte, gerundete Form besitzen, um die Wand des physiologischen Kanals nicht zu verletzen.

4. Dehnbare Hülse gemäß Anspruch 3, **gekennzeichnet dadurch, dass** die hakenförmigen Gebilde aus vorstehenden Elementen (7) bestehen, die an den Enden und/oder im Hauptteil der Hülse (1) angeordnet sind.

5. Dehnbare Hülse gemäß Anspruch 3, **gekennzeichnet dadurch, dass** die hakenförmigen Gebilde vorspringende, einziehbare Elemente (8) sind, die von den nach innen gebogenen Blättern (9) der Hülse (1) getragen werden, so dass sie normalerweise hinter der Wand des rohrförmigen Elements liegen und erst dann hervortreten, wenn der Ballon (2) mit Druck beaufschlagt wird.

6. Dehnbare Hülse nach einem der Ansprüche 4 und 5, **gekennzeichnet dadurch, dass** die vorstehenden Elemente (7, 8) durch das Falten von Wandpartien mit geeigneter Form der Hülse (1) realisiert werden.

7. Dehnbare Hülse nach einem der Ansprüche 4 bis 6, **gekennzeichnet dadurch, dass** die vorstehenden Elemente (7, 8) abnehmbar sind.

8. Dehnbare Hülse nach Anspruch 3, **gekennzeichnet dadurch dass** die hakenförmigen Gebilde aus Rillen (10) oder ringförmigen Wellen in der Hülsenwand (1) bestehen.

9. Dehnbare Hülse nach einem der vorstehenden Ansprüche, **gekennzeichnet dadurch dass** sie aus mehreren miteinander gelenkig verbundenen Elementen (1', 1") besteht, wobei jeweils zwei aufeinanderfolgende Elemente durch ein auf ihnen in Längsrichtung befestigtes Band (13) verbunden sind.

10. Dehnbare Hülse gemäß Anspruch 9, **gekennzeichnet dadurch, dass** jedes Gelenk zwischen den gelenkig verbundenen Elementen (1', 1") mit Hilfe von abnehmbaren Haken (14) blockiert oder gelöst werden kann.

11. Dehnbare Hülse gemäß einem der Ansprüche 1 bis 8, **gekennzeichnet dadurch, dass** sie aus mehreren gelenkig miteinander verbundenen Elementen (1', 1") besteht, wobei jeweils zwei aneinander grenzende Elemente durch ein Netz (15) oder eine weiche Folie verbunden sind.

12. Dehnbare Hülse gemäß einem der vorstehenden Ansprüche, **gekennzeichnet dadurch, dass** die Innenwand der Hülse (1) von einem weichen Schlauch (11) bedeckt ist, der ein dichtendes Futter darstellt.

13. Dehnbare Hülse gemäß einem der Ansprüche 1 bis 11, **gekennzeichnet dadurch, dass** die Außenseite der Hülse (1) von einem weichen Schlauch (11) bedeckt ist, der ein dichtendes Futter darstellt.

14. Dehnbare Hülse nach einem der Ansprüche 12 oder 13, **gekennzeichnet dadurch, dass** der weiche Schlauch (11) aus gewebten oder gewirkten synthetischen Fasern besteht.

15. Dehnbare Hülse nach einem der Ansprüche 12 bis 14, **gekennzeichnet dadurch, dass** der weiche Schlauch (11) aus einem der folgenden Werkstoffe besteht: Polyester (Typ "Dacron"), Silikonfasern (Typ "Teflon"), Polyurethan oder Polyethylen.

16. Dehnbare Hülse nach einem der vorstehenden Ansprüche 13 bis 15, **gekennzeichnet dadurch, dass** der weiche Schlauch (11) Öffnungen besitzt, um den Übergang zu sekundären Kanälen zu ermöglichen.

17. Dehnbare Hülse nach einem der vorstehenden Ansprüche, **gekennzeichnet dadurch, dass** sie aus Edelstahl ist und Punkte (16), längsseitige (17) oder ringförmige Bänder (18) besitzt, die aus Schichten eines im Röntgenbild sichtbaren, auf elektrolytischem Wege aufgebrachten Schwermetalls bestehen, wie zum Beispiel Tantal, Titan oder Gold, wobei die Schichtdicke etwa 0,1 Millimeter beträgt.
